**Europäisches Patentamt**

**European Patent Office**

**Office européen des brevets**

(19)

(11) Veröffentlichungsnummer: **0 012 360**
**B1**

(12) **EUROPÄISCHE PATENTSCHRIFT**

(45) Veröffentlichungstag der Patentschrift:
**12.10.83**

(51) Int. Cl.³: **A 61 B 17/04**

(21) Anmeldenummer: **79104949.7**

(22) Anmeldetag: **05.12.79**

(54) **Knopf für chirurgische Zwecke.**

(30) Priorität: **09.12.78 DE 2853289**

(43) Veröffentlichungstag der Anmeldung:
**25.06.80 Patentblatt 80/13**

(45) Bekanntmachung des Hinweises auf die Patenterteilung:
**12.10.83 Patentblatt 83/41**

(84) Benannte Vertragsstaaten:
**AT BE CH DE FR GB IT LU NL SE**

(56) Entgegenhaltungen:
**DE-B-2157529**
**US-A-2075508**
**US-A-2199025**
**US-A-3541591**
**US-A-3910281**
**US-A-3976079**

(73) Patentinhaber: **Intermedicat GmbH, Gerliswilstrasse 45, CH-6020 Emmenbrücke (CH)**

(72) Erfinder: **Fuchs, Heinz, Pfleffrain 22, D-3508 Melsungen (DE)**
Erfinder: **Müller, Hans-Friedrich, Dr., Bredensbergweg 37, D-2000 Hamburg 92 (DE)**
Erfinder: **Hecht, Hildegard, Zur Schönen Aussicht 1, D-3509 Spangenberg (DE)**

(74) Vertreter: **von Kreisler, Alek, Dipl.-Chem. et al, Deichmannhaus am Hauptbahnhof, D-5000 Köln 1 (DE)**

Knopf für chirurgische Zwecke

Die Erfindung bezieht sich auf einen Knopf für chirurgische Zwecke, bestehend aus einer als parallelwandiges Gehäuse gestalteten Scheibe, in der ein Schlitz ausgebildet ist, der einen zentralen axialen Durchlass zum Hindurchführen eines Fadens mit dem Scheibenumfang verbindet und der von einem parallel zur Scheibe schwenkbaren Scheibensegment zum Festklemmen des Fadens zwischen den Gehäusewänden und Flächen des Scheibensegmentes verschlossen wird.

Beim chirurgischen Wundverschluss ist es häufig erforderlich, Nahtmaterialfäden, die unter einer mehr oder weniger starken Zugspannung stehen, auf der Haut zu verankern. Das Verankerungsmittel muss ohne Schaden für die Haut bis zu einigen Wochen lang an Ort und Stelle bleiben können und soll die Zugkräfte aufnehmen, die in unterschiedlicher und wechselnder Stärke auf diese Fäden einwirken, so dass eine Entlastung der Wundfläche herbeigeführt wird. Die Spannungsfreiheit des Gewebes an der Wundnaht ist eine wichtige Voraussetzung für die einwandfreie Verheilung von Gewebe, weil Spannung des Gewebes längs der Verschlusslinie einer Wunde die ständige Gefahr bedeutet, dass die Wunde mechanisch wieder aufbricht. Ausserdem stört Spannung den Heilprozess selbst. Eine einfache Knopfnaht kann die erforderliche Spannungsfreiheit des Gewebes oft nicht bewirken, weil sie infolge der geringen Auflagefläche des Fadens auf der Haut die Gefahr einer Drucknekrose bedingen würde. Zur Vergrösserung der Auflagefläche und somit zur Entlastung der Haut werden Bauschnähte, Platten oder Knöpfe verwendet. Bei Bauschnähten muss der Faden um Bäusche geschlungen und verknotet werden. Dies ist umständlich. Im Falle der bekannten Platten und Knöpfe ist zusätzlich zur Verknotung jeweils erforderlich, dass der Faden durch axiale Durchlässe gefädelt werden muss, was anwendungstechnisch für den Chirurgen umständlich und zeitraubend ist. Bei der Fixierung des durch einen Knopf hindurchgefädelten Fadens mittels Bleikugeln kommt nachteilig hinzu, dass hier eine sichere Verankerung des Fadens nicht gewährleistet ist. Durch das Einfädeln und Verknoten des Fadens ist es ausserdem nur schwer möglich, von vornherein die richtige Fadenspannung einzustellen, so dass sich evtl. der Faden allmählich lockert und nachgespannt werden müsste. Dies ist jedoch bei einem verknoteten Faden praktisch nicht möglich.

Ein Knopf, bei dem das Verknoten des Fadens dadurch entfällt, dass ein Klemmteil vorgesehen ist, der den Faden an dem Knopf fixiert, ist in der US-A-2075508 beschrieben. Hierbei ist auf eine tellerförmige Scheibe eine geschlitzte Scheibe oder ein Streifen als Klemmteil aufgenietet. Der Niet ist axial durchlocht, so dass durch den Knopf ein Faden geführt werden kann, der zwischen der tellerförmigen Scheibe und dem Klemmteil festklemmbar ist. Damit hat auch dieser Knopf den Nachteil, dass der Faden umständlich in den Knopf eingefädelt werden muss. Ausserdem sind sowohl die Einstellung der richtigen Fadenspannung als auch die Nachstellung der Fadenspannung problematisch.

Bei einem durch die DE-B2-2157529 bekannten Knopf ergeben sich im wesentlichen die gleichen Probleme. Dieser Knopf ist so ausgebildet, dass ein das axiale Scheibenloch zum Hindurchführen des Fadens schneidendes radiales Loch vorgesehen ist, in das ein Stift derart einschiebbar ist, dass er den durch das axiale Loch geführten Faden zwischen sich und der Lochwand festklemmt. Es hat sich gezeigt, dass die Handhabung auch dieses Knopfes umständlich ist und grosse Geschicklichkeit des Chirurgen erfordert, weil auch hierbei der Faden eingefädelt werden muss. Die Fixierung des Fadens mittels des Stiftes ist nicht zuverlässig sicher und eine Korrektur der Fadenspannung ist nicht möglich, weil sich der Stift nicht mehr erfassen und aus dem radialen Loch herausziehen lässt.

Eine Möglichkeit zur Erleichterung der Einführung des Fadens in den axialen Durchlass der Scheibe und seines Festklemmens in diesem Durchlass ist in der US-A-3976079 beschrieben. Hierbei ist in der Scheibe ein Schlitz ausgebildet, der den zentralen axialen Durchlass mit dem Scheibenumfang verbindet, so dass der Faden vom Scheibenumfang her radial in den Durchlass eingeführt werden kann und ein axiales Einfädeln entfällt. Als Klemmittel dient eine um eine Achse quer zur Scheibenebene schwenkbare Klinke, die mit einem gezahnten Teil den Faden gegen eine innere Wand der Scheibe drückt. Die innere Wand verläuft gerade und setzt einen Rand des Durchlasses axial fort. Der festgeklemmte Faden verläuft geradlinig und im Bereich der Klemmzone umlenkungsfrei durch die Scheibe, so dass er nur auf einem kurzen Stück klemmend gehalten ist. Um trotzdem eine Verschiebungssicherung des Fadens zu erreichen, ist die Zahnung an der Klinke notwendig. Diese ist jedoch ungünstig, weil die Zähne bei geschlossener Klinke den Faden beschädigen oder sogar durchtrennen können, woraus sich ein Verlust oder eine Minderung der Reissfestigkeit oder bei Trennung das Versagen der Befestigung überhaupt ergibt. Ausserdem ist es nachteilig, dass das Klemmittel nicht arretierbar ist und dadurch ein unbeabsichtigtes Lösen des Fadens möglich wird. Diese Gefahr wird dadurch vergrössert, dass ein flacher und schmaler Griffteil der Klinke über die Scheibenebene vorsteht, so dass über den Knopf hinweggleitendes Material sich an dem Griffteil festhaken und die Klinke auslösen kann. Bei geschlossener Klinke bleibt der radiale Schlitz der Scheibe offen. Hierdurch kann sich die Scheibe in Verbandsmaterial od. dgl. verhaken, was ebenfalls bezüglich der Sicherheit der Fadenfestlegung problematisch ist.

Ferner ist der eingangs erwähnte Knopf für eine Vorrichtung zur Befestigung eines auf einer Schnur abgehefteten Papierstapels bekannt (GB-A-569957), der aus einer als parallelwandiges Gehäuse gestalteten Scheibe besteht, in der ein Schlitz ausgebildet ist, der einen zentralen axialen Durchlass zum Hindurchführen eines Fadens mit dem Scheibenumfang verbindet, und der von einem parallel zur Scheibe schwenkbaren Scheibensegment zum Festklemmen des Fadens zwischen den Gehäusewänden und Flächen des Scheibensegmentes verschlossen wird. Bei diesem Knopf fehlt eine Verriegelung des Scheibensegmentes in seiner Endstellung innerhalb des Gehäuses und es wird lediglich durch Verkeilung zwischen Faden und Gehäusewand in Stellung gehalten. Dies mag für den Zweck der Zusammenheftung von Papierblättern ausreichen. Für chirurgische Zwecke genügt es nicht. Zusätzlich zur Sicherheit ist bei einem chirurgischen Knopf die Einfachheit der Handhabung wesentlich und unter diesem Gesichtspunkt ist bei dem bekannten Knopf auch nachteilig, dass nicht nur ein radialer Schlitz in beiden Wänden des Gehäuses ausgebildet, sondern ausserdem der Randsteg des Scheibensegmentes an einer Stelle durchbrochen ist, damit der Faden radial in den zentralen Durchlass eingeführt werden kann. Dies erfordert eine sehr genaue gegenseitige Ausrichtung von Schlitz und Durchbrechung des Scheibensegmentes, die jedoch unmöglich ist, weil das Scheibensegment durch die Gehäusewände verdeckt wird. Ausserdem muss der Faden sehr genau in den zentralen Durchlass der Scheibe eingeführt werden, da er anderenfalls von dem Scheibensegment nicht ordnungsgemäss festgeklemmt bzw. sogar in den offenen Schlitz hineingeschoben wird, aus dem er dann herausgleitet. Es ist vorgesehen, dass der Arm des Scheibensegmentes an seinem Ende einmal umgebogen ist, und dass dieser umgebogene Teil in eine Kerbe in der einen Wand des Gehäuses eingreift, wenn das Scheibensegment seine Klemmstellung eingenommen hat. Da eine Sicherung des Scheibensegmentes in dieser Stellung fehlt, wäre bei Verwendung des Knopfes für chirurgische Zwecke zu befürchten, dass das Ende des Armes während der Liegezeit des Knopfes an einer Wundnaht über den Umfang des Knopfes vortritt, so dass über den Knopf hinweggleitendes Material, z. B. Verbandsmaterial od. dgl., sich an dem Arm festhakt und das Scheibensegment aus dem Gehäuse herauszieht. Im gleichen Moment wäre das Fadenende frei und dies hätte für die Wundheilung schwerwiegende Folgen. Da im übrigen der radiale Schlitz in beiden Wänden des Gehäuses immer über den grössten Teil seiner Länge offen bleibt, kann sich auch in dem Schlitz Verbandsmaterial od. dgl. verhaken, was ebenfalls bezüglich der Sicherheit der Fadenfestlegung problematisch ist.

Der Erfindung liegt die Aufgabe zugrunde, einen Knopf für chirurgische Zwecke so auszubilden, dass er sich einfach handhaben lässt und nach seiner Festlegung auf einem Faden seine Klemmstellung zuverlässig beibehält.

Diese Aufgabe wird dadurch gelöst, dass das Scheibensegment von dem offenen Ende des Schlitzes her über den Schlitz hinweg bis in eine Endstellung beweglich ist, in der sein innerer Randteil hinter dem Durchlass liegt und das Scheibensegment mit der Scheibe mittels einer Rastvorrichtung verriegelt ist.

Ein solcher Knopf lässt sich einfach und unkompliziert handhaben, weil der Faden einfach in die Öffnung des Schlitzes eingelegt und durch seitlichen Druck auf das Scheibensegment von seinem inneren Randteil durch den Schlitz in den axialen Durchlass der Scheibe zwangsläufig mitgenommen wird. Es entfällt damit jeglicher Zeitaufwand zum Einführen des Fadens in den Durchlass. Die Fadeneinführung lässt sich mit einer Hand bewerkstelligen. Der Chirurg kann beim Ansetzen des Knopfes mit der einen Hand zwanglos die Fadenspannung kontrollieren und mit der anderen Hand den Knopf in der gewünschten Position plazieren sowie an dem Faden festklemmen. Durch die selbsttätige Verriegelung des Scheibensegmentes in der Endstellung nimmt es immer zwangsläufig seine Stellung mit maximaler Klemmwirkung für den Faden ein, ohne dass der Chirurg hierauf besonders achten muss. In der Endstellung hat der innere Randteil den Faden hinter den Durchlass gedrückt, und er wird abgewinkelt festgeklemmt, ohne abgeschert oder beschädigt zu werden. Dabei ist die durch den weit in das Gehäuse hineinragenden Rand des Scheibensegmentes gebildete Fadenschleife sehr lang und dies hat zur Folge, dass der Faden auf einem langen Stück eingeklemmt gehalten ist, wodurch eine grössere Reibung und damit eine höhere Zugfestigkeit sowie insbesondere Rutschsicherheit erzielt werden. Die zuverlässige Festklemmung des Fadens gewährleistet die für eine einwandfreie Verheilung von Gewebe erforderliche Spannungsfreiheit des Gewebes an der Wundnaht.

Da das Scheibensegment sich von der offenen Seite des Schlitzes her über diesen gegen den Durchlass in der Scheibe bewegt, wird nicht nur der Faden mitgenommen und in den Durchlass zuverlässig eingeführt, sondern es wird ausserdem der gesamte Schlitz von dem Scheibensegment abgedeckt und verschlossen, wenn dieses sich in der verriegelten Endstellung befindet. In dem Schlitz kann sich daher Verbandsmaterial oder dergleichen nicht verhaken und gemeinsam mit der Verriegelung des Scheibensegmentes garantiert dies die Sicherheit des Verschlusses des Knopfes.

Bei der Handhabung des Knopfes kann entweder der zu fixierende Faden durch den Schlitz in den gegen ihn offenen Durchlass seitlich hineingezogen werden oder es kann die Scheibe von der Seite her auf den Faden aufgeschoben werden. Schlitz und Durchlass sind bei geöffnetem Knopf immer frei zugänglich, und es ist dem Chirurgen möglich, den Knopf in Anpassung an die jeweiligen Gegebenheiten unterschiedlich zu handhaben.

In vorteilhafter Ausgestaltung der Erfindung ist vorgesehen, dass in der einen Wand des Gehäuses der Durchlass und der Schlitz ausgebildet sind und die andere Gehäusewand bis zum Durchlass zurückgesetzt ist, und dass der innere Randteil des Scheibensegmentes in den Hohlraum des Gehäuses hinter den Rand der zurückgesetzten Gehäusewand hineinragt und in verriegelter Endstellung eine hinter dem inneren Randteil vorgesehene Absatzkante des Scheibensegmentes wenigstens teilweise gegen den Rand der zurückgesetzten Gehäusewand anliegt. Diese Ausgestaltung hat den Vorteil, dass das Scheibensegment bei seiner Bewegung relativ zur Scheibe verkantungsfrei in dem Gehäuse geführt ist, und dass der Faden beim Eindringen des Scheibensegmentes in das Gehäuse mehrmals abgewinkelt und auf beiden Seiten des Scheibensegmentes zwischen einer Scheibensegmentfläche und einer Gehäusewand eingeklemmt wird. Hierdurch ergibt sich die hervorragende rutschsichere Festklemmung des Fadens in dem Knopf. Eine zusätzliche Klemmwirkung wird zwischen den aneinanderliegenden Rändern der zurückgesetzten Gehäusewand und der Absatzkante des Scheibensegmentes auf den Faden ausgeübt. Falls dies nicht gewünscht wird, kann der Rand der zurückgesetzten Gehäusewand eine mit dem Durchlass fluchtende Aussparung aufweisen, durch die der Faden hindurchtritt ohne zwischen die Absatzkante des Scheibensegmentes und den Rand der zurückgesetzten Wand eingeklemmt zu sein.

Der innere Randteil des Scheibensegmentes kann gerade gestaltet und gezahnt ausgebildet sein. Die Zähne des Randteiles verhindern, dass der Faden während des Einklemmvorganges seitlich verrutschen kann. Alternativ kann der innere Randteil des Scheibensegmentes halbkreisförmig gewölbt ausgebildet sein. Bei dieser Ausgestaltung wird der Umlenkungsweg des Fadens innerhalb des Gehäusehohlraumes der Scheibe besonders lang, und die auf den Faden wirkende Reibung wird vergrössert.

Zur Verriegelung der Scheibe und des Scheibensegmentes in der Endstellung dient eine Rastvorrichtung. Diese besteht aus zusammengreifenden Haken, wobei der eine Haken an einem elastischen Arm vorgesehen ist und eine über den Aussenumfang der Scheibe vorstehende Nase aufweist. Die über den Aussenumfang der Scheibe vorstehende Nase gestattet eine einfache Entriegelung der Rastvorrichtung zur Öffnung des Knopfes und Aufhebung der Einklemmung des Fadens. Auf diese Weise ist eine eventuelle Korrektur der Fadenspannung durch stärkeres Anspannen oder Nachlassen der Fadenspannung jederzeit möglich. Dem anderen Haken kann eine über den Aussenumfang der Scheibe vorstehende Nase zugeordnet sein. Bei verriegelter Rastvorrichtung sind die beiden Nasen zueinander so ausgerichtet, dass ihr Zusammendrücken zwischen Daumen und Zeigefinger einer Hand eine Freigabe des Hakenzusammengriffs bewirkt und der Klemmschlitz zwischen Scheibe und Scheibensegment sich öffnet. Der Knopf kann mit einer Hand geöffnet oder geschlossen werden. Die andere Hand ist frei zur Handhabung des Fadens.

Vorteilhafterweise ergänzen sich bei Zusammengriff der Haken der Rastvorrichtung die Aussenkonturen der Scheibe und des Scheibensegmentes zu einem symmetrischen Körper. Der Körper kann jede beliebige abgerundete Form aufweisen. Vorzugsweise ist er kreisscheibenförmig. Er ist in verschiedenen Grössen, je nach Fadenstärke und zu erwartender Zugspannung herstellbar. Flache, glatte Konturen garantieren eine gute Adaptierung an die Hautoberfläche sowie an den Verband, weil kein Auftragen des Verbandes eintritt. Der Knopf ist universell einsetzbar für alle Stütznähte, zum Beispiel bei Bauchdeckennähten (Ventrofil), bei Plastiken, bei Sehnennähten nach Lengemann, Intracutannähten usw.

Der Knopf wird insgesamt aus einem Kunststoffmaterial hergestellt, das eine reizlose gute Hautverträglichkeit bietet. Gegebenenfalls kann eine Unterfütterung mit Textilstoff oder einer Schaumgummiplatte vorgesehen sein.

Ausführungsbeispiele sind in der Zeichnung schematisch dargestellt.

Es zeigt:

Fig. 1 eine schaubildliche Ansicht des geschlossenen Knopfes;

Fig. 2 eine schaubildliche Ansicht des geöffneten Knopfes;

Fig. 3 einen Querschnitt des geschlossenen Knopfes nach Fig. 1;

Fig. 4 eine schaubildliche Ansicht einer anderen Ausführungsform eines geschlossenen Knopfes;

Fig. 5 eine schaubildliche Ansicht des geöffneten Knopfes nach Fig. 4, und

Fig. 6 einen Querschnitt des Knopfes nach Fig. 4.

Der Knopf für die Festlegung chirurgischen Nahtmaterials ist als Kreisscheibenkörper mit glatten Flächen ausgebildet, der im wesentlichen aus einer Scheibe 1 und einem Scheibensegment 2 besteht, die mittels eines Filmscharnieres 3 miteinander verbunden sind. Sowohl die Scheibe 1 als auch das Scheibensegment 2 und das Filmscharnier 3 sind zweckmässig aus Kunststoff gefertigt.

Die Scheibe 1 ist als parallelwandiges Gehäuse ausgebildet, dessen eine Wand 4 einen Schlitz 7 aufweist, dessen geschlossenes Ende 5 einen Durchlass für einen Faden 6 bildet. Die andere Wand 8 ist bis zum Durchlass 5 zurückgesetzt. Sie trägt an einem Ende einen Arm 9, an den ein nach innen gerichteter Haken 10 angeformt ist. An dem Haken 10 befindet sich eine über den Aussenumfang der Scheibe 1 vorstehende Nase 11.

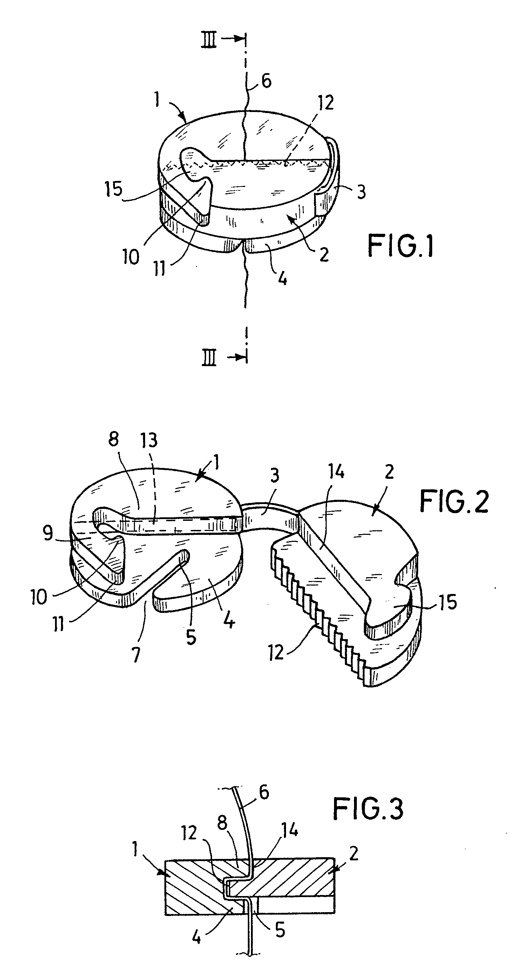

Das Scheibensegment 2 ragt mit einem inneren Randteil 12 in den Hohlraum 13 des Gehäuses hinein und befindet sich in geschlossenem Zustand des Knopfes hinter dem Durchlass 5 in der Wand 4 des Gehäuses der Scheibe 1. Der gerade Rand 12 kann gezahnt sein, um ein seitliches Verschieben des Fadens zu verhindern. In geschlossener Stellung des Knopfes kommt eine Absatzkante 14 des Scheibensegmentes 2 gegen den Rand der Wand 8 der Scheibe zur Anlage und ein nach aussen gerichteter Haken 15 an dem Scheibensegment hintergreift den Haken 10 der Scheibe 1 nach der Art einer Rastvorrichtung. Zum Lösen der Rastverriegelung zwischen den beiden Haken 10 und 15 drückt man gegen die Nase 11 des Hakens 10, woraufhin das Scheibensegment 2 aus dem Zusammengriff mit der Scheibe 1 freikommt (Fig. 2).

Die Scheibe 1 und das Scheibensegment 2 wirken nach der Art von Klemmbacken zusammen, zwischen denen der Faden 6 umgelenkt und festgeklemmt wird. Der Faden 6 wird bei geöffnetem Knopf 1 von der Seite her durch den Schlitz 7 in den Durchlass 5 eingeführt. Sodann wird durch seitlichen Andruck gegen das Scheibensegment 2 der Faden 6 von dem inneren Rand 12 in den Gehäusehohlraum 13 mitgenommen. In verriegeltem Zustand des Knopfes ist der Faden mehrmals umgelenkt und mehrmals zwischen zusammenwirkenden Klemmflächen eingeklemmt, wie Fig. 3 zeigt. Ein Verrutschen ist praktisch ausgeschlossen.

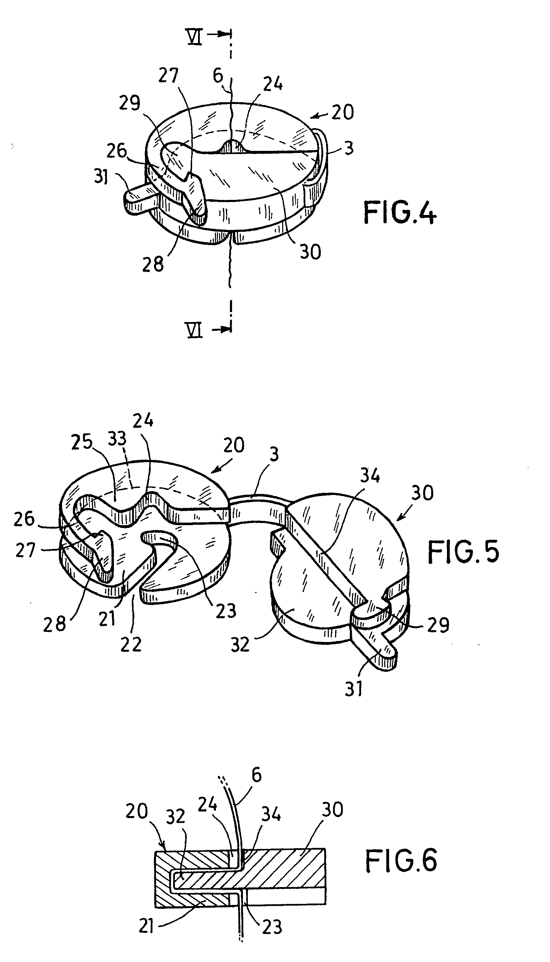

Das zweite Beispiel nach den Fig. 4 bis 6 entspricht funktionsmässig im wesentlichen dem Knopf gemäss Fig. 1 bis 3, jedoch ist seine Gestaltung etwas abgewandelt.

Die Scheibe 20 weist in ihrer Wand 21 einen nach aussen abgerundet verbreiterten Schlitz 22 auf, der in einen abgewinkelten Durchlass 23 für einen Faden übergeht. Der Durchlass 23 fluchtet im wesentlichen mit einer Aussparung 24 in der anderen Wand 25 der Scheibe 20. Ein leicht gebogener Arm 26 der Wand 25 trägt einen nach innen gerichteten Haken 27, an den eine nach aussen ragende Nase 28 angeformt ist.

Mit dem Haken 27 greift ein nach aussen gerichteter Haken 29 eines Scheibensegmentes 30 zusammen. Auch dem Haken 29 ist eine nach aussen gerichtete Nase 31 zugeordnet, die sich in verriegeltem Zustand des Knopfes in Schliessbewegungsrichtung des Scheibensegmentes 30 hinter der Nase 8 befindet (Fig. 4). Diese Ausbildung hat zur Folge, dass die beiden Haken 27 und 29 durch Zusammendrücken der Nasen 28 und 21 zwischen Daumen und Zeigefinger voneinander getrennt werden, so dass der Knopf sich mit einer Hand öffnen lässt.

Der innere Randteil 32 des Scheibensegmentes 30 verläuft bei diesem Beispiel halbkreisförmig gewölbt. Entsprechend ist der Hohlraum 33 der Scheibe 20 ausgebildet.

Der gewölbte innere Randteil 32 kann glattrandig sein, weil ein seitliches Verrutschen des Fadens durch die Aussparung 24 in der Wand 25 der Scheibe 20 verhindert wird.

Der Faden 6 wird von dem halbkreisförmig gewölbten Teil des Scheibensegmentes 30 weit in den Hohlraum 33 der Scheibe 20 hineingeführt. Durch die vergrösserte Fadenschlaufe ist die Länge des zwischen den Gehäusewänden 21 und 25 sowie dem Scheibensegmentteil eingeklemmten Fadenstückes grösser als bei dem Beispiel der Fig. 1–3. Die grössere eingeklemmte Fadenlänge vermittelt einen verschiebungssicheren Halt des Knopfes auf dem Faden, obwohl der Rand der zurückgesetzten Wand 25 im Bereich des Fadendurchtritts ausgespart ist und an dieser Stelle gegen die Absatzkante 34 des Scheibensegmentes 30 nicht anliegt, so dass auf den Faden hier keine zusätzliche Klemmwirkung ausgeübt wird.

**Patentansprüche**

1. Knopf für chirurgische Zwecke, bestehend aus einer als parallelwandiges Gehäuse gestalteten Scheibe (1), in der ein Schlitz (7) ausgebildet ist, der einen zentralen axialen Durchlass (5) zum Hindurchführen eines Fadens mit dem Scheibenumfang verbindet und der von einem parallel zur Scheibe (1) schwenkbaren Scheibensegment (2) zum Festklemmen des Fadens zwischen den Gehäusewänden und Flächen des Scheibensegmentes (2) verschlossen wird, dadurch gekennzeichnet, dass das Scheibensegment (2; 30) von dem offenen Ende des Schlitzes (7; 22) her über den Schlitz (7; 22) hinweg bis in eine Endstellung beweglich ist, in der sein innerer Randteil (12; 32) hinter dem Durchlass (5; 23) liegt und das Scheibensegment (2; 30) mit der Scheibe (1; 20) mittels einer Rastvorrichtung (10, 15; 27, 29) verriegelt ist.

2. Knopf nach Anspruch 1, dadurch gekennzeichnet, dass in der einen Wand (4; 21) des Gehäuses der Durchlass (5; 23) und der Schlitz (7; 22) ausgebildet sind und die andere Gehäusewand (8; 25) bis zum Durchlass (5; 23) zurückgesetzt ist, und dass der innere Randteil (12; 32) des Scheibensegmentes (2; 30) in den Hohlraum (13; 33) des Gehäuses hinter dem Rand der zurückgesetzten Gehäusewand (8; 25) hineinragt und in verriegelter Endstellung eine hinter dem inneren Randteil (12; 32) vorgesehene Absatzkante (14; 34) des Scheibensegmentes (2; 30) wenigstens teilweise gegen den Rand der zurückgesetzten Gehäusewand (8; 25) anliegt.

3. Knopf nach Anspruch 1 oder 2, dadurch gekennzeichnet, dass der Rand der zurückgesetzten Gehäusewand (25) eine mit dem Durchlass (23) fluchtende Aussparung (24) aufweist.

4. Knopf nach einem der Ansprüche 1 bis 3, dadurch gekennzeichnet, dass der innere Randteil (12) des Scheibensegmentes (2) gerade gestaltet und gezahnt ausgebildet ist.

5. Knopf nach einem der Ansprüche 1 bis 3, dadurch gekennzeichnet, dass der innere Randteil (32) des Scheibensegmentes (30) halbkreisförmig gewölbt ausgebildet ist.

6. Knopf nach einem der Ansprüche 1 bis 5, dadurch gekennzeichnet, dass die Scheibe (1; 20) mit dem Scheibensegment (2; 30) über ein Filmscharnier (3) verbunden ist.

7. Knopf nach einem der Ansprüche 1 bis 6, dadurch gekennzeichnet, dass die Rastvorrichtung aus zusammengreifenden Haken (10, 15; 27, 29) gebildet ist, und dass der eine Haken (10; 27) an einem elastischen Arm (9; 26) vorgesehen ist und eine über den Aussenumfang der Scheibe (1; 20) vorstehende Nase (11; 28) aufweist.

8. Knopf nach Anspruch 7, dadurch gekennzeichnet, dass dem anderen Haken (29) eine über den Aussenumfang der Scheibe (20) vorstehende Nase (31) zugeordnet ist.

9. Knopf nach einem der Ansprüche 1 bis 8, dadurch gekennzeichnet, dass bei Zusammengriff der Haken (10, 15; 27, 29) der Rastvorrichtung die Aussenkonturen der Scheibe (1; 20) und des Scheibensegmentes (2; 30) sich zu einem symmetrischen Körper ergänzen.

### Claims

1. Button for surgical purposes comprising a disk (1) designed as a parallel-walled housing and including a slot (7) which connects a central axial passage (5) to the circumference of the disk to guide a thread therethrough and which is closable by a disk segment (2) swivelling in parallel to the disk (1) for clamping the treated between the housing walls and faces of the disk segment (2), characterized in that the disk segment (2; 30) is movable from the open end of the slot (7; 22) over the slot (7; 22) to a final position in which its inner marginal part (12; 32) is situated past the passage (5; 23) and that the disk segment (2; 30) is latched with the disk (1; 20) by means of a click-stop device (10, 15; 27, 29).

2. Button according to claim 1, characterized in that the passage (5; 23) and the slot (7; 22) are made in one wall (4; 21) of the housing while the other housing wall (8; 25) is recessed to the passage (5; 23), and that the inner marginal part (12; 32) of the disk segment (2; 30) protrudes into the cavity (13, 33) of the housing past the edge of the recessed housing wall (8; 25) and that in latched final position, a recessed edge (14, 34) of the disk segment (2; 30) provided behind the inner marginal part (12, 32) adjoins at least partly the edge of the recessed housing wall (8; 25).

3. Button according to claim 1 or 2, characterized in that the edge of the recessed housing wall (25) comprises a recess (24) in alignment with the passage (23).

4. Button according to any one of claims 1 to 3, characterized in that the inner marginal part (12) of the disk segment (2) is of rectilinear shape and toothed.

5. Button according to any one of claims 1 to 3, characterized in that the inner marginal part (32) of the disk segment (30) is semicircularly arcuate.

6. Button according to any one of claims 1 to 5, characterized in that the disk (1; 20) is connected to the disk segment (2; 30) via a flexible hinge (3).

7. Button according to any one of claims 1 to 6, characterized in that the click-stop device comprises hooks (10, 15; 27, 29) gripping with each other, with one hook (10, 27) being provided on a flexible arm (9; 26) and comprising a nose (11; 28) protruding over the outer circumference of the disk (1; 20).

8. Button according to claim 7, characterized in that to the other hook (29), a nose (31) is coordinated that protrudes over the outer circumference of the disk (20).

9. Button according to any one of claims 1 to 8, characterized in that with latching engagement of the hooks (10, 15; 27, 29) of the click-stop device, the outer contours of the disk (1; 20) and of the disk segment (2; 30) form a symmetrical body.

### Revendications

1. Bouton à usage chirurgical consistant en un disque qui se présente comme un boîtier à parois parallèles (1) dans lequel est formée une fente (7) reliant un passage axial central (5) au pourtour du disque pour l'introduction du fil, et qui est fermé par un segment de disque (2) pivotant parallèlement au disque (1) afin de coincer solidement le fil entre les parois du boîtier et les faces du segment de disque (2), caractérisé en ce que le segment de disque (2; 30) peut se déplacer à partir de l'extrémité ouverte de la fente (7; 22), en passant au-dessus de la fente (7; 22) jusqu'à une position finale dans laquelle sa partie marginale interne (12; 32) se trouve au-delà du passage (5; 23), et ou le segment de disque (2; 30) est verrouillé sur le disque (1; 20) au moyen d'un dispositif d'arrêt (10, 15; 27, 29).

2. Bouton selon la revendication 1, caractérisé en ce que le passage (5; 23) et la fente (7; 22) sont pratiqués dans l'une des parois (4; 21) du boîtier et l'autre paroi du boîtier (8; 25) est en retrait jusqu'au passage (5; 23), et en ce que la partie marginale interne (12; 32) du segment de disque (2; 30) pénètre dans la cavité (13; 33) du boîtier au-delà du bord de la paroi du boîtier en retrait (8; 25), et que dans la position verrouillée finale un bord décalé (14; 34) du segment de disque (2; 30) prévu en arrière de la partie marginale interne (12; 32) s'ajuste au moins partiellement contre le bord de la paroi en retrait du boîtier (8; 25).

3. Bouton selon la revendication 1 ou 2, cractérisé en ce que le bord de la paroi du boîtier en retrait (25) présente une échancrure (24) en alignement avec le passage (23).

4. Bouton selon l'une quelconque des revendications 1 à 3, caractérisé en ce que le bord interne (12) du segment de disque (2) est de forme rectiligne et est dentelé.

5. Bouton selon l'une quelconque des revendications 1 à 3, caractérisé en ce que le bord interne (32) du segment de disque (30) a une forme cintrée semi-circulaire.

6. Bouton selon l'une quelconque des revendications 1 à 5, caractérisé en ce que le disque (1; 20) est relié au segment de disque (2; 30) par l'intermédiaire d'un film formant charnière (3).

7. Bouton selon l'une quelconque des revendications 1 à 6, caractérisé en ce que le dispositif d'arrêt est formé de crochets (10, 15; 27, 29) s'assemblant l'un avec l'autre et en ce que l'un des crochets (10; 27) est adapté sur un bras élastique (9; 26) et présente en ergot (11; 28) faisant saillie au-delà de la périphérie du disque (1; 20).

8. Bouton selon la revendication 7, caractérisé en ce qu'un ergot (31) faisant saillie au-delà de la périphérie du disque (20) est attaché à l'autre crochet (29).

9. Bouton selon l'une quelconque des revendications 1 à 8, caractérisé en ce que lors de l'assemblage des crochets (10, 15; 27, 29) du dispositif d'arrêt, les contours extérieurs du disque (1; 20) et du segment de disque (2; 30) se complètent pour former un corps symétrique.

FIG.1

FIG.2

FIG.3

0 012 360

FIG.4

FIG.5

FIG.6

9